# EUROPÄISCHE PATENTSCHRIFT

(11) **EP 1 281 319 B1**
(45) Veröffentlichungstag und Bekanntmachung des Hinweises auf die Patenterteilung: **28.09.2005**
(21) Anmeldenummer: 02016685.6
(22) Anmeldetag: 26.07.2002
(51) Int. Cl.: A01N 25/04, A01N 31/02, A61K 7/48

(54) **Alkoholische Gele**
Alcoholic gels
Gels alcooliques

(30) Priorität: 04.08.2001 DE 10138456
(43) Veröffentlichungstag der Anmeldung: 05.02.2003
(73) Patentinhaber: Bode Chemie GmbH & Co., 22525 Hamburg (DE)
(72) Erfinder: Seibt, Horst, Dr., 10245 Berlin (DE); Ballschuh, Detlef, Dr., 12524 Berlin (DE); Knieler, Roland, Dr., 21227 Benderstorf (DE); Rudolf, Marco, Dr., 22299 Hamburg (DE); Pietsch, Hanns, Dr., 20148 Hamburg (DE)
(74) Vertreter: Rabanus, Birgit

(56) Entgegenhaltungen:
- EP-A- 0 689 767
- WO-A-02/15852
- WO-A-97/00667
- US-A- 2 861 060
- US-A- 5 385 736
- DATABASE WPI Week 198639 Derwent Publications Ltd., London, GB; AN 1986-255375 XP002215356 & JP 61 183224 A (KAO CORP.), 15. August 1986 (1986-08-15)

## Beschreibung

Die vorliegende Erfindung betrifft alkoholische Gele zur Desinfektion der Hände und Haut im Haushalt, in der Industrie und im Krankenhaus. Insbesondere betrifft die Erfindung Gele mit einem hohen Alkoholgehalt ("hochprozentige Gele") sowie wasserfreie Gele.

Alkoholische Gele sind an sich bekannt. Sie werden zum Einreiben des Körpers z. B. als "Franzbranntwein-Gel", als Fußpflegepräparate oder als Hände- oder Hautdesinfektionsmittel verwendet. Die meisten der im Handel befindlichen Gele, die zur Händedesinfektion vorgesehen sind, enthalten 60 bis zu maximal 70 Vol.-% Alkohol. Als Gelbildner werden üblicherweise Polyacrylsäuren verwendet, die mit organischen Aminen neutralisiert werden. Solche dem Stand der Technik entsprechenden Neutralisationsmittel werden z. B. in der Firmenschrift der Fa. Goodrich empfohlen:

| **Alkoholgehalt** | **Gelbildner** | **Neutralisationsmittel** |
|---|---|---|
| bis 20 Vol.-% Alkohol | Polyacrylsäure (Carbopol) | Natriumhydroxyd |
| bis 30 Vol.-% Alkohol | Polyacrylsäure (Carbopol) | Kaliumhydroxyd |
| bis 60 Vol.-% Alkohol | Polyacrylsäure (Carbopol) | Triethanolamin |
| bis 60 Vol.-% Alkohol | Polyacrylsäure (Carbopol) | Trisamino* |
| bis 80 Vol.-% Alkohol | Polyacrylsäure (Carbopol) | Aminopropylmethan |
| bis 90 Vol.-% Alkohol | Polyacrylsäure (Carbopol) | Neutrol TE ** |
| bis 90 Vol.-% Alkohol | Polyacrylsäure (Carbopol) | Diisopropanolamin |
| bis 90 Vol.-% Alkohol | Polyacrylsäure (Carbopol) | Triisopropanolamin |
| bis 90 Vol.-% Alkohol | Polyacrylsäure (Carbopol) | Ethomeen C-25 *** |

| | | |
|---|---|---|
| * 2-Amino-2-(Hydroxymethyl)-1,3-Propandiol | | |
| ** Tetrahydroxypropylethylendiamin | | |
| *** Ethoxyliertes Cocosamin (15 EO) | | |

In der Patentliteratur werden eine Reihe von alkoholischen Gelen beschrieben, die zur Händedesinfektion vorgesehen sind:

Die US 4,695,453 (Henkel Corp.) beansprucht alkoholische Gele mit 40 bis 50 Gew.-% Ethanol, 20 bis 30 Gew.-% Isopropanol, 0,5 bis 2 Gew.-% Benzylalkohol verdickt mit Poly(2-acrylamido-2-methylpropan-sulfonsäure) oder mit Poly(ammonium-2-acrylamido-2-methylpropansulfonat).

In der US Patentschrift 4,478,853 (S.C. Johnson & Son) werden alkoholische Hautpflegelotionen beansprucht, die 35 bis 50 Vol.-% Alkohol enthalten. Das Verdickersystem besteht aus vemetzter Polyacrylsäure, zum Neutralisieren wird Triethanolamin, Triethylamin, Isopropylamin, Kalium- oder Natriumhydroxyd verwendet.

In der US-Patentschrift 4,956,170 (Johnson & Johnson) werden alkoholische Gele beansprucht, die einen Gehalt von 60 bis 75 Gew.-% Ethanol und/oder Isopropanol aufweisen; als Verdicker werden 0,4 bis 2 Gew.-% einer vernetzten Polyacrylsäure in Verbindung mit einem Neutralisationsmittel beansprucht. Neutralisationsmittel können z. B. ethoxylierte Alkylamine (C₁₀₋₁₈ - Alkyl mit 5 bis 25 Ethoxy-Einheiten) sein. Diese Gele enthalten zusätzlich hautpflegende kosmetische Substanzen.

Die US-Patentschrift 5,167,950 (S.C. Johnson & Son) beansprucht eine alkoholische antimikrobielle Creme, die als Schaum aufgetragen wird, bestehend aus 52 bis 75 Gew.-% Alkohol; bevorzugter Gelbildner ist vernetzte Polyacrylsäure, es können aber auch Cellulosederivate verwendet werden. Zum Neutralisieren werden Triethanolamin, Natriumhydroxid, Monoethanolamin, Diethylstearylamin, Diethanolamin, Dipropanolamin sowie ethoxylierte Fettsäureamine mit einem Alkylrest von 10 bis 18 Kohlenstoffatomen eingesetzt.

Die Zubereitungen, die in den obengenannten Schutzrechten beschrieben werden, sollen als Händedesinfektionsmittel geeignet sein und beispielsweise im Krankenhaus verwendet werden. Allerdings wurde die mikrobizide Wirksamkeit nicht überprüft, sondern wegen des hohen Alkoholgehaltes vorausgesetzt.

Die europäische Patentschrift EP 0 223 681 (Calgon Corporation) beansprucht Gele auf der Basis von 30 bis 90 Gew.-% Alkohol; das Verdickersystem ist Hydroxypropylcellulose. Die mikrobizide Wirksamkeit wurde an Schweinehäuten getestet.

Die europäische Patentschrift EP 0 689 767 (Becton, Dickinson and Comp.) beansprucht alkoholische Formulierungen für die Hautdesinfektion mit einem Alkoholgehalt von 50 bis 80 Gew.-%, einem Polyalkylanoxiddimethylsiloxanblockcopolymer, wobei die Zubereitungen auch - beispielsweise mit Hilfe von Polyoxypropylenpolyoxyethylenkondensaten, Hydroxypropylcellulosen oder Carbopolymeren - verdickt werden können.

Die Europäische Patentanmeldung EP 0 930 065 (Ethicon Inc.) beansprucht Gele mit mehr als 40 Vol.-% Alkohol (insbesondere 40 bis 80 Vol.-%), welche vernetzte Polyacrylsäure als Verdicker sowie quarternäre Ammoniumverbindungen als antimikrobielle Wirkstoffe enthalten.

Die US Patentschrift 5,512,199 (Bacton, Dickinson And Comp.) beansprucht ein alkoholisches Reinigungsgel bestehend aus 50 bis 80 Gew.-% Alkohol und Chitosan als Verdicker.

Die Patentanmeldung WO 97/00667 (3M) beschreibt alkoholische Gele mit mehr als 35 Gew.-% Alkohol, welche für die chirurgische Händedesinfektion geeignet sind. Das Verdickersystem besteht aus zwei Emulgatoren und erfordert keinen zusätzlichen Verdicker.

Die US-amerikanische Patentschrift 5,908,619 (3M) beschreibt alkoholische Gele mit mindestens 20 Gew.-% Alkohol, einem Verdickersystem bestehend aus einem anionischen oder kationischem Polymer und einem entgegengesetzt geladenen Tensid mit mindestens 16 C-Atomen im Alkylrest.

Tabellarische Übersicht über den Stand der Technik:

| Tabellarische Übersicht über den Stand der Technik: | | | |
|---|---|---|---|
| **Patentnr.** | **Alkohol** | **Verdicker** | **Zusatz- Wirkstoff** |
| US 4 695 453 | 40 - 50 Gew.-% Ethanol, | Polyacrylamidomethyl- | |
| | 20 - 30 Gew.-% Isopropanol, | propansulfonsäure oder | |
| | 0,5 - Gew.-% Benzylalkohol | Polyammoniumacrylamidomethylpropansulfonat | |
| US 4 478 853 | 35 - 50 Vol.-% Alkohole | Polyacrylsäure | - |
| US 4 956 170 | 60 - 75 Gew.-% Alkohole | Polyacrylsäure | - |
| US 5 167 950 | 52 - 75 Gew.-% Alkohole | Polyacrylsäure | - |
| EP 223 681 | 30 - 90 Gew.-% Alkohole | Hydroxypropylcellulose | - |
| EP 689 767 | 50 - 80 Gew.-% Alkohole | diverse | - |
| EP 930 065 | 40 - 80 Vol.-% Alkohol | Polyacrylsäure | QAV |
| US 5 512 199 | 50 - 80 Gew.-% Alkohole | Chitosan | - |
| WO 97/00667 | > 35 Gew.-% Alkohole | gelad. Polymer | - |
| US 5 908 619 | > 20 Gew.-% Alkohole | gelad. Polymer | alle |

Die Gele entsprechend dem Stand der Technik weisen einige, zum Teil erhebliche Mängel auf. So ergeben insbesondere Gele mit dem Verdicker Hydroxypropylcellulose nach dem Abdunsten des Alkohols klebrige und schmierige Rückstände, die beim Verreiben der Hände unästhetische "schmutzige" Klümpchen ("Rubbel", englisch: balling) bilden. Ähnliches gilt für Gele, welche mit Chitosan oder auch Polyamidosulfonsäure verdickt sind.

Ferner neigen auch Gele, welche auf der Basis von Polyacrylsäure verdickt werden, zur Bildung klebriger Schichten und zur Bildung von Rückständen (Rubbel). Dies ist um so mehr der Fall je mehr Verdicker (polymerer Bestandteil der Verdickerkompositon) verwendet wird.

Die Menge an Verdicker hängt vom Typ des Polymeren, z. B. der Polyacrylsäure, ab: die verschiedenen Typen unterscheiden sich durch das Molekulargewicht, den Verzweigungsgrad und durch die Art und Menge des verwendeten Comonomeren. Die benötigte Verdickermenge hängt außerdem von der Höhe der Alkoholkonzentration ab: je höher die Konzentration an Alkohol, um so mehr Verdicker benötigt man. Die Menge des benötigten Verdickers hängt ferner von der Höhe der erwünschten Viskosität ab.

Bei Verwendung von Polyacrylsäure als Verdicker können die Nachteile der Klebrigkeit und der Bildung von Rückständen zum Teil durch die Verwendung von Siliconölen (beispielsweise durch Cyclomethicon) kompensiert werden.

Ein weiterer wesentlicher Nachteil der nach dem Stand der Technik hergestellten alkoholischen Gele ist eine nur unzureichende desinfizierende Wirksamkeit. Die mikrobiozide Wirksamkeit desinfizierender Zubereitungen kann auf verschiedene Weisen nachgewiesen werden. In einigen der zitierten Patentschriften wird die mikrobizide Wirksamkeit der beanspruchten Gele angenommen, weil die entsprechenden Alkohole (Ethanol, Propanol-1 und Propanol-2) unverdickt bekanntermaßen mikrobizid wirksam sind. In anderen wird die mikrobizide Wirksamkeit an Hand von minimalen Hemmhofkonzentrationen nachgewiesen.

Nach dem neuesten Stand der Technik wird die Eignung eines alkoholischen Händedesinfektionsmittels für die hygienische Händedesinfektion nach der Europa-Norm EN 1500 nachgewiesen. Sehr ähnlich ist der Test Hygienische Händedesinfektion" der deutschen Gesellschaft für Hygiene und Mikrobiologie (DGHM). Die Eignung für die chirurgische Händedesinfektion läßt sich mit Hilfe des Tests pr EN 12 791 nachweisen.

Es hat sich herausgestellt, daß alkoholische Gele des Standes der Technik z. B. den Test nach EN 1500 nicht bestehen, obwohl sie einen Alkoholgehalt aufweisen, der in flüssigen, nicht viskosen Zubereitungen üblicherweise wirksam ist. Solche Gele sind für eine Verwendung im Krankenhaus nicht ausreichend geeignet, bestenfalls eignen sie sich für die Verwendung im Haushalt oder die allgemeine Hygiene.

US 5,385,736 offenbart ein transdermales Drug-Delivery-System zur Behandlung und Prophylaxe von Migräne, welches ein klebend beschichtetes Folienmaterial umfasst. In den Beispielen 3, 4 und 5 enthält die klebende Schicht unter anderem 1 Gew.-% N,N-Dimethyldodecylamin-N-oxid und 60 Gew.-% (74 : 6 : 20)-Copolymerisat von Isooctylacrylat : Acrylamid : Vinylacetat. Es war somit Aufgabe der Erfindung, alkoholische Gele zu entwickeln, die nach EN 1500 wirksam sind, beim Einreiben in die Hände ein angenehmes Hautgefühl ergeben, in den Händen nicht schmierig und nicht klebrig wirken sowie beim Einreiben keine Abriebpartikel entstehen lassen. Eine weitere Aufgabe der vorliegenden Erfindung war es, ein möglichst gut hautverträgliches Verdickersystem zu verwenden und basische Neutralisationsmittel als Coverdicker zu vermeiden.

Es war überraschend und für den Fachmann nicht vorherzusehen, daß alkoholische Desinfektionsgele, dadurch gekennzeichnet, daß sie ein Verdickungssystem enthalten, welches aus mindestens einer verzweigten Polyacrylsäure und einem und mehreren Aminoxiden der allgemeine Strukturformel I 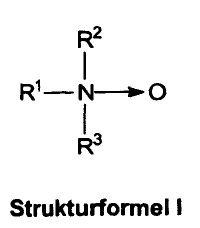 in der R¹, R² und R³ unabhängig voneinander einen aliphatischen Alkyl-, Isoalkyl- oder Alkenylrest, einen alicyclischen tertiären Alkyl- oder Amidoalkyl-Rest mit jeweils 1 bis 22 Kohlenstoffatomen oder eine Oxyalkylgruppe der Formel -(CₙH₂ₙO)ₓH bedeuten, wobei n = 2 oder 3 und x gleich Zahlen von 1 bis 25 sind, als Co-Verdicker besteht,
wobei der oder die Alkohole gewählt werden aus der Gruppe enthaltend Ethanol, Propanol-1 und Propanol-2, welche entweder einzeln oder in beliebigen Mischungen miteinander eingesetzt werden, und
wobei die Gele frei sind von dem (74: 6 :20)-Copolymerisat von Isooctylacrylat : Acrylamid : Vinylacetat,
den Nachteilen des Standes der Technik abhelfen würden.

Es war ferner überraschend und nicht vorhersehbar, dass die nachstehend ausführlich beschriebenen Aminoxide als Coverdicker in Kombination mit verzweigter Polyacrylsäure in alkoholischen Gelen auch bei Abwesenheit von Wasser wirksam sind, wobei die Gele frei von dem (74: 6 :20)-Copolymerisat von Isooctylacrylat: Acrylamid : Vinylacetat sind.

Die erfindungsgemäßen Gele können vorteilhaft - wenngleich nicht zwingend - weitere nichtflüchtige antimikrobielle Wirkstoffe enthalten. Die erfindungsgemäßen alkoholischen Gele sind ab einem Alkoholgehalt von 80 Vol.-% besonders wirksam gegen Mikroorganismen und erfüllen die Anforderungen der Europanorm EN 1500: "Hygienische Händedesinfektion". Unterhalb 80 % sind sie für hygienische Händemaßnahmen im Haushalt, in der Lebensmittel- oder Pharmaindustrie geeignet.

Die erfindungsgemäßen Gele sind wegen ihres günstigen pH-Wertes besonders hautfreundlich. Die Gele im Sinne der vorliegenden Erfindung können vorteilhaft kosmetisch wirksame Bestandteile enthalten, die z. B. die Hautfreundlichkeit der Zubereitungen weiter verbessern.

Erfindungsgemäß vorteilhaft enthalten die alkoholischen Gele mehr als 30 Gew.-%, vorzugsweise 80 bis 100 Gew.-% eines oder mehrerer Alkohole, welche gewählt sind aus der Gruppe enthaltend Ethanol, Propanol-1 und Propanol-2, wobei die Alkohole entweder allein oder im Gemisch eingesetzt werden können.

Als Verdickersysteme dienen verzweigte Polyacrylsäuren und ein oder mehrere Aminoxide der allgemeinen Strukturformel (I). Für eine Viskositätserhöhung sind Co-Verdicker erforderlich, da sich mit Polyacrylsäure allein keine ausreichende Viskositätserhöhung erreichen läßt, um aus Alkoholen ein Gel zu erhalten.

Es war insbesondere überraschend, daß sich die Aminoxide als Co-Verdicker zum Verdicken eines niederen Alkohols oder Alkoholgemisches mit verzweigter Polyacrylsäure eignen, da hierzu nach dem Stand der Technik üblicherweise bisher ein entgegengesetzt geladenes Neutralisationsmittel benötigt wurde. Aminoxide zählen zu den nichtionogenen Stoffen. Die erfindungsgemäß eingesetzten Aminoxide sind dementsprechend keine Neutralisationsmittel im herkömmlichen Sinn, und es war daher überraschend und nicht vorhersehbar, daß die erfindungsgemäßen Aminoxide in Verbindung mit Polyacrylsäure alkoholische Systeme verdicken würden.

Die erfindungsgemäßen Aminoxide sind "Coverdicker" im Sinne der vorliegenden Erfindung, welche insbesondere in ihrer Hautverträglichkeit den Aminen überlegen sind.

Die besonderen Vorteile dieses Verdickungssystems aus verzweigter Polyacrylsäure und Aminoxid-Coverdicker liegt darin, daß alkoholische Gele über den ganzen Konzentrationsbereich von 30 bis 100 Gew.-% Alkoholgehalt stabil sind.

Es ist ein weiterer Vorteil der erfindungsgemäßen alkoholischen Gele mit dem Verdickersystem Polyacrylsäure und Aminoxid, daß auch bei hohen Alkoholkonzentrationen über 80 Gew.-% nur wenig Verdicker benötigt wird.

Die Verbindungstypen der Aminoxide, die sich für den Einsatz als Coverdicker eignen, sind unterschiedlicher Natur; es können tensidische aber auch nichttensidische Aminoxide sein.

Nichttensidische Aminoxide, wie beispielsweise Tris(hydroxyethyl)-aminoxid (1), Dimethylhydroxyethyl-aminoxid (2), Dimethyl-2-hydroxypropyl-aminoxid (3) oder Methyl-morpholinaminoxid (4), 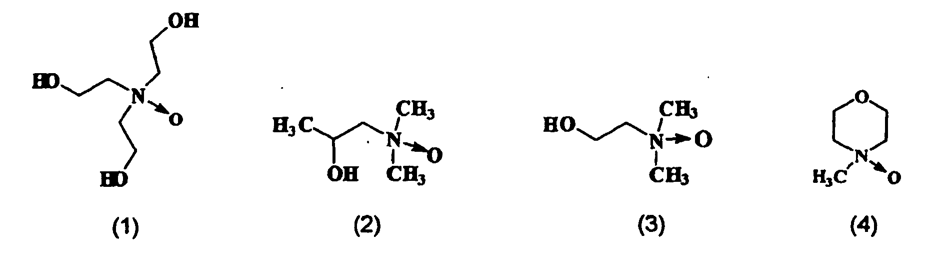 bilden vorzugsweise erfindungsgemäße alkoholische Gele mit einem Alkoholgehalt von weniger als 50 Gew.-% (vgl. Beispiele 6 bis 9; 11 und 12).

Kurzkettige bzw. mittelkettige tensidische Aminoxide, wie beispielsweise Dimethyl-alkyl- (C₆ bis C₁₄) -aminoxide (5) oder als Sonderfall einer großräumigen Substituentenanordnung das Trioctylaminoxid (6), können vorteilhaft im Sinne der vorliegenden Erfindung zur Herstellung alkoholischer Gele mit einem Alkoholgehalt von 35 bis 90 Gew.-% eingesetzt werden (vgl. Beispiele 10; 13 bis 19).

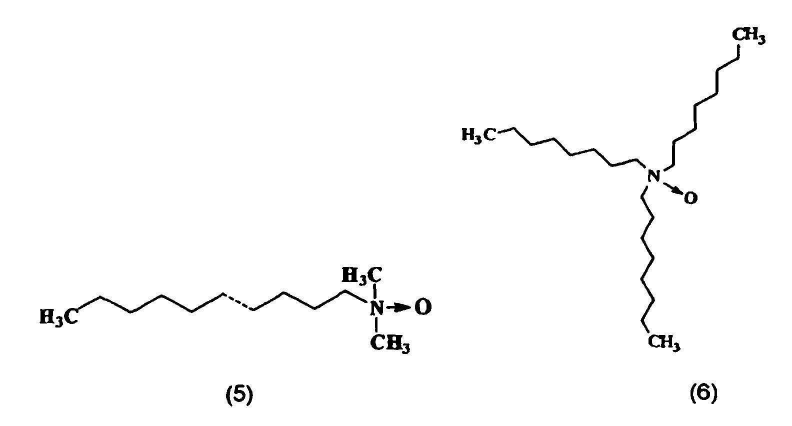

Erfindungsgemäß vorteilhafte Alkylaminoxide, welche sich besonders gut zur Herstellung alkoholischer Gele mit einem Alkoholgehalt von mehr als 90 Gew.-% eignen, sind ethoxylierte oder propoxylierte bzw. ethoxy-propoxylierte mittel- bis langkettige tensidische Alkylaminoxide der allgemeinen Formel I, in der R¹ Alkylreste mit 8 bis 22 Kohlenstoffatomen, R² und R³ gleich oder verschieden eine Oxyalkylgruppe der Formel -(CₙH₂ₙO)ₓH bedeuten, wobei n gleich 2 oder 3 und x gleich Zahlen von 1 bis 25 sind.

Besonders vorteilhaft sind Stearyl-heptaoxyethylenaminoxid (7) 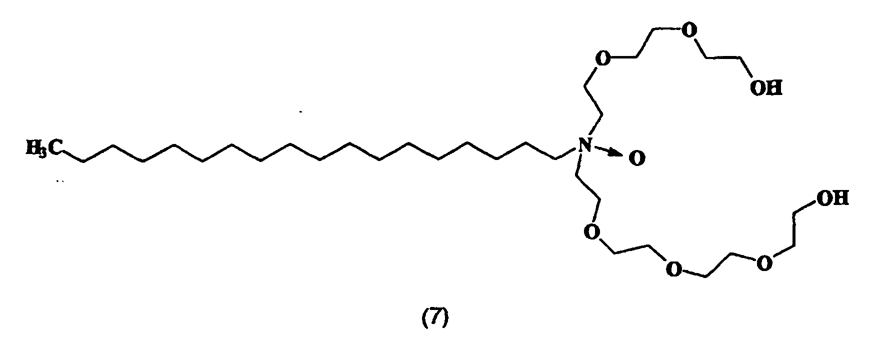 bzw. Stearyl-decaoxypropylenaminoxid (8)

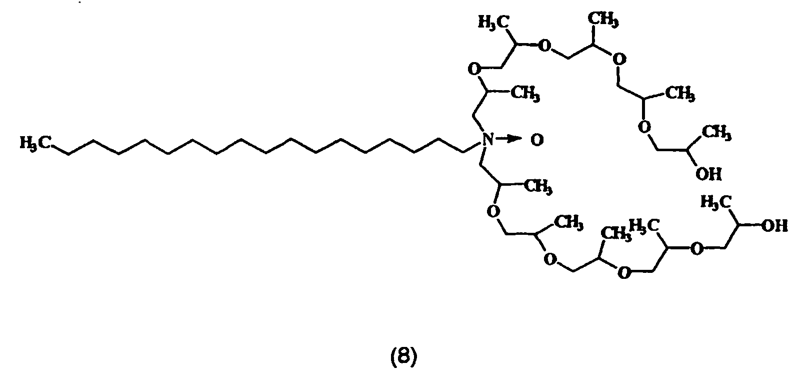

Bei Verwendung von propoxylierten Alkyl(C₁₈)-aminoxiden (8) mit 5 bis 15 Propylenoxid-Einheiten in alkoholischen Gelen mit einem Alkoholgehalt > 90 Gew.-% (Ethanol, Propanol-1, Propanol-2 oder Gemische derselben) erhält man Rezepturen, die besonders gute Gebrauchswerteigenschaften aufweisen (vgl. Beispiele 1 - 5). Ganz besonders vorteilhaft im Sinne der vorliegenden Erfindung ist Stearyl-decaoxypropylenaminoxid (8).

Die mit allen Aminoxidtypen der allgemeinen Formel (I) gebildeten alkoholischen Gele sind stabile Systeme, deren Strukturen sich nach längerem Stehen weiter verfestigen.

Überraschend ist darüber hinaus, daß die den Aminoxiden zu Grunde liegenden tertiären Amine (wie z. B. Dimethylfettalkylamine, Trioctylamin, Dimethylhydroxyethylamin oder Dimethyl-2-hydroxypropylamin oder Methylmorpholin) mit Ausnahme der ethoxylierten bzw. propoxylierten Amine, nicht befähigt dazu sind, alkoholische Gele mit verzweigter Polyacrylsäure aufzubauen.

Bei Verwendung von Tris(hydroxyethyl)aminoxid (1) oder den ethoxylierten (7) sowie propoxylierten (8) Aminoxiden in Kombination mit der verzweigten Polyacrylsäure zur Herstellung von alkoholischen Gelen werden stabilere Strukturen erhalten, als durch Einsatz der entsprechenden tertiären Amine (wie Triethanolamin) oder den ethoxylierten sowie propoxylierten Aminen möglich wäre.

Sehr wesentlich für das Hautgefühl der gelartigen Zubereitungen ist die absolute Menge an Verdickersystem (bestehend aus verzweigter Polyacrylsäure und Aminoxid). Da diese Verbindungen nicht verdunsten und auch nicht in die Haut einziehen, bildet sich auf der Haut eine Schicht, die um so größer ist, je öfter man sich hintereinander die Hände desinfiziert, und je höher der Anteil an Verdickersystem im Produkt ist. Diese Schicht kann einen klebrigen oder schmierigen Eindruck auf der Haut hinterlassen. Deshalb sollte der Anteil an Verdickersystem so niedrig wie möglich sein.

Der Anteil des Verdickersystems (bestehend aus verzweigter Polyacrylsäure und Aminoxid) wird vorzugsweise aus dem Bereich von 0,5 bis 5,0 Gew.-% gewählt, bezogen auf das Gesamtgewicht der Zusammensetzung. Es war insbesondere überraschend, daß die erfindungsgemäßen Gele auch mit einem Wassergehalt von weniger als 5 Gew.-%, sogar weniger als 1 Gew.-% ausreichend verdickbar und stabil sind.

Es ist erfindungsgemäß vorteilhaft, das Gewichtsverhältnis von Polyacrylsäure zu Co-Verdicker aus dem Bereich von 1 : 1 bis 1 : 3 zu wählen.

Zusätzlich enthalten die erfindungsgemäßen alkoholischen Gele bevorzugt hautpflegende Substanzen, gegebenenfalls ferner Farbstoff und Parfüm. Die erfindungsgemäßen alkoholischen Gele können 0,1 bis 2,5 % Gew.-% Hautpflegestoffe enthalten. Diese Hautpflegestoffe werden beispielsweise gewählt aus:
- der Gruppe der feuchtigkeitsspendende Substanzen, wie beispielsweise Glycerol, Pyrrolidoncarbonsäure usw.
- der Gruppe der rückfettenden Substanzen, wie Tetradecanol, Hexadecanol, Paraffinöl usw.
- der Gruppe der antiklebende Substanzen, wie Siliconöle, vorzugsweise Cyclomethicone usw.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In den nachfolgenden Beispielen wurde die antimikrobielle Wirksamkeit nach EN 1500 überprüft, die dermatologischen Eigenschaften wurden über Fragebögen beim Test nach EN 1500 von den Probanden abgefragt.

### Beispiele:

### Beispiele 1 - 5

Es wurden alkoholische Händedesinfektionsmittel folgender Zusammensetzung durch Zusammenrühren der Bestandteile bei Raumtemperatur hergestellt:

| **Beispiel** | **1** | **2** | **3** | **4** | **5** |
|---|---|---|---|---|---|
| Ethanol¹ | 97 | 96 | 96 | 76,8 | 76,8 |
| Carbopol ETD 2020 | 0,35 | 0,7 | 0,54 | 0,7 | 0,54 |
| Aminoxid² | 0,71 | 1,42 | 1,06 | 1,42 | 1,06 |
| Hautschutzlösung³ | 0,5 | 0,5 | 0,5 | 0,5 | 0,5 |
| Wasser | 0,146 | 1,38 | 1,9 | 0,58 | 1,1 |
| Propan-1-ol | - | - | - | 20 | 20 |

| | | | | | |
|---|---|---|---|---|---|
| ¹ Ethanol 99 Gew.-% vergällt mit 1 Gew.-% Butanon-2, | | | | | |
| ² Stearylaminoxid^{*)}, propoxyliert mit 10 Propylenoxideinheiten (gemäß Formel (8)), 70 Gew.-%, gelöst in Wasser (21,9 Gew.-%) und Ethanol 8,1 Gew.-%) | | | | | |
| ³ Gemisch aus 36,36 Gew.-% Heptamethylnonan, 36,36 Gew.-% 2-Ethylhexyl-2-ethyl-hexanoat, 9,1 Gew.-% Lanolinalkohol und 18,18 Gew.-% Tetradecanol. *⁾ Das als Rohstoff verwendete Stearylamin hatte folgende Kohlenstoffkettenverteilung: 3 % C₁₄; 29 % C₁₆; 63 % C₁₈; ≤ 3 % C₂₀. | | | | | |

Von diesen Gelen wurden die Viskositäten mit Hilfe eines Rotationsviskosimeters "Brookfield DV II+" gemessen. Die Drehzahl betrug 10 U/Min, das Drehmoment betrug 905 dyn x cm.
Das Hautgefühl und die Akzeptanz der Präparate wurden von den Probanden während der Tests nach EN 1500 abgefragt. Gefragt wurde: Wie bewerten Sie das Hautgefühl beim Einreiben des Produktes" (sehr unangenehm, unangenehm, angenehm, sehr angenehm), "Bemerken Sie in der Trocknungsphase ein Klebrigkeitsgefühl?" (klebt sehr stark, klebt, ganz leichtes Kleben, klebt nicht), "Rubbelt das Produkt auf der Haut?" (rubbelt sehr stark, rubbelt, rubbelt gelegentlich, rubbelt nicht), "Welches Hautgefühl haben Sie nach dem Trocknen?" (sehr unangenehm, unangenehm, angenehm, sehr angenehm), "Sind Ihre Hände nach 30 Sekunden Trocken?" (noch sehr feucht, noch etwas feucht, fast trocken, trocken).

| **Ergebnisse:** | | | | | |
|---|---|---|---|---|---|
| | **Beispiel 1** | **Beispiel 2** | **Beispiel 3** | **Beispiel 4** | **Beispiel 5** |
| Viskosität (mPas) | 5040 | 7600 | 7200 | 5100 | 3200 |
| Hautgefühl beim Einreiben | Sehr angenehm | angenehm | angenehm | angenehm | angenehm |
| Klebrigkeitsgefühl in der Trocknungsphase | Klebt nicht | Leichtes Kleben | Klebt nicht | Klebt nicht | Klebt nicht |
| Rubbeln | Kein | Kein | Kein | Kein | Kein |
| | Rubbeln | Rubbeln | Rubbeln | Rubbeln | Rubbeln |
| Trocken nach 30 s | trocken | trocken | trocken | fast trocken | trocken |

Antimikrobielle Wirksamkeit nach EN 1500: Alle Präparate waren signifikant besser als 60 Vol.-% Propanol-2.

### Beispiel 6

Bestandteile:
30 % Ethanol (99.9 %)
0,3 % Carbopol ETD 2020
1,8 % Dimethyl-2-hydroxypropyl-aminoxid (gemäß Formel (2), 30 %-ig, gelöst in Wasser)
0,5 % Hautschutzlösung (Zusammensetzung siehe Beispiel 1 bis 5)
67,4 % Wasser
Viskosität: 6380 mPas

### Beispiel 7

Bestandteile:
30 % Ethanol (99,9 %)
0,3 % Carbopol ETD 2020
1,2 % Tri(hydroxyethyl)aminoxid (gemäß Formel (1), 50 %-ig, gelöst in Ethanol (25%) und Wasser (25 %))
0,5 % Hautschutzlösung (Zusammensetzung siehe Beispiel 1 bis 5)
68,0 % Wasser
Viskosität: 5580 mPas

### Beispiel 8

Bestandteile:
30 % Ethanol (99,9 %)
0,3 % Carbopol ETD 2020
1,4 % Dimethyl-hydroxyethylaminoxid (gemäß Formel (3), 45 %-ig, gelöst in Wasser) 0,5% Hautschutzlösung (Zusammensetzung siehe Beispiel 1 bis 5)
67,8% Wasser
Viskosität: 6720 mPas

### Beispiel 9

Bestandteile:
35 % Ethanol (99,9 %)
0,3 % Carbopol ETD 2020
1,2 % 4-Methyl-morpholin-4-oxid (gemäß Formel (4)), 50 %-ig, gelöst in Wasser
0,5 % Hautschutzlösung (Zusammensetzung siehe Beispiel 1 bis 5)
63,0 % Wasser
Viskosität: 6550 mPas

### Beispiel 10

Bestandteile:
35 % Ethanol (99,9 %)
0,3 % Carbopol ETD 2020
30 % Dimethyl-hexylaminoxid (1,5 %-ig gelöst in Wasser)
0,5 % Hautschutzlösung (Zusammensetzung siehe Beispiel 1 bis 5)
34,2 % Wasser
Viskosität: 7020 mPas

### Beispiel 11

Bestandteile:
40 % Ethanol (99,9 %)
0,3 % Carbopol ETD 2020
1,2 % Tri(hydroxyethyl)aminoxid (50 Gew.-%, gelöst in Ethanol (25 Gew.-%) und Wasser (25 Gew.-%)
0,5 % Hautschutzlösung (Zusammensetzung siehe Beispiel 1 bis 5)
58,0 % Wasser
Viskosität: 5650 mPas

### Beispiel 12

Bestandteile:
60% Ethanol (99,9 %)
0,3% Carbopol ETD 2020
1,2 % Tri(hydroxyethyl)aminoxid (50 Gew.-%, gelöst in Ethanol (25 Gew.-%) und Wasser (25 Gew.-%)
0,5% Hautschutzlösung (Zusammensetzung siehe Beispiel 1 bis 5)
38,0% Wasser
Viskosität: 4580 mPas

### Beispiel 13

Bestandteile:
60% Ethanol (99,9%)
0,3% Carbopol ETD 2020
0,8% Trioctylaminoxid (50 Gew.-%, gelöst in Ethanol (25 Gew.-%) und Wasser (25 Gew.-%)
0,5% Hautschutzlösung (Zusammensetzung siehe Beispiel 1 bis 5)
38,4% Wasser
Viskosität: 5720 mPas

### Beispiel 14

Bestandteile:
45% Propanol-2
30% Propanol-1
0,35 % Carbopol ETD 2020
0,9% Trioctylaminoxid (50 Gew.-%, gelöst in Ethanol (25 Gew.-%) und Wasser (25 Gew.-%)
0,5% Hautschutzlösung (Zusammensetzung siehe Beispiel 1 bis 5)
23,25% Wasser
Viskosität: 5250 mPas

### Beispiel 15

Bestandteile:
45% Propanol-2
30% Propanol-1
0,35% Carbopol ETD 2020
20,0% Decyldimethylaminoxid (2,0 %-ig, gelöst in Wasser)
0,5 % Hautschutzlösung (Zusammensetzung siehe Beispiel 1 bis 5)
4,15% Wasser
Viskosität 4810 mPas

### Beispiel 16

Bestandteile:
45% Propanol-2
30% Propanol-1
0,35% Carbopol ETD 2020
1,4% Dimethyl-dodecylaminoxid (30 %-ig, gelöst in Wasser)
0,5% Hautschutzlösung (Zusammensetzung siehe Beispiel 1 bis 5) 22,75% Wasser
Viskosität: 5020 mPas

### Beispiel 17

Bestandteile:
95% Ethanol (99,9 %)
0,3% Carbopol ETD 2020
1,4% Dimethyl-dodecylaminoxid (30 %-ig, gelöst in Wasser)
0,5% Hautschutzlösung (Zusammensetzung siehe Beispiel 1 bis 5) 2,8 % Wasser
Viskosität: 4670 mPas

### Beispiel 18

Bestandteile:
94,5% Ethanol (99,9%)
0,3% Carbopol ETD 2020
1,4% Dimethyl-tetradecylaminoxid (30 %-ig), gelöst in Ethanol (40 %) und Wasser (30 %)
0,5% Hautschutzlösung (Zusammensetzung siehe Beispiel 1 bis 5)
3,3% Wasser
Viskosität: 4360 mPas

### Beispiel 19

Bestandteile:
45% Propanol-2
30% Propanol-1
0,35 % Carbopol ETD 2020
1,5% Dimethyl-tetradecylaminoxid (30 %-ig), gelöst in Ethanol (40 %) und Wasser (30 %)
0,5% Hautschutzlösung (Zusammensetzung siehe Beispiel 1 bis 5)
22,65% Wasser
Viskosität: 5230 mPas

### Beispiel 20

Bestandteile:
45% Propanol-2
30% Propanol-1
0,35% Carbopol ETD 2020
1,5% Kokosbis(2-hydroxyethyl)aminoxid (30 Gew.-%, gelöst in Wasser)
0,5 % Hautschutzlösung (Zusammensetzung siehe Beispiel 1 bis 5)
22,65% Wasser
Viskosität: 5490 mPas

### Beispiel 21

Bestandteile:
45% Propanol-2
30% Propanol-1
0,35% Carbopol ETD 2020
0,75% ethoxyliertes Oleylaminoxid^{*)} mit 5 EO-Einheiten (66,8 %-ig), gelöst in 13,3 % Wasser und 19,9 % Ethanol)
0,5% Hautschutzlösung (Zusammensetzung siehe Beispiel 1 bis 5)
23,4% Wasser
Viskosität: 4770 mPas

^{*)} Das als Rohstoff verwendete Oleylamin hatte folgende Kohlenstoffkettenverteilung: 1 % C₁₂; 4% C₁₄; 12% C₁₆; ~ 83% C₁₈ ungesättigt.

### Beispiel 22

Bestandteile:
95% Ethanol (99,9%)
0,3% Carbopol ETD 2020
0,625% ethoxyliertes Stearylaminoxid^{*)} mit 7 EO-Einheiten (80 %-ig), gelöst in 13,3 % Wasser und 6,7 % Ethanol)
0,5% Hautschutzlösung (Zusammensetzung siehe Beispiel 1 bis 5)
2,8% Wasser
Viskosität: 6230 mPas

^{*)} Das als Rohstoff verwendete Stearylamin hatte folgende Kohlenstoffkettenverteilung: 3% C₁₄; 29% C₁₆; 63% C₁₈; ≤ 3% C₂₀.

### Beispiel 23

Bestandteile:
95% Ethanol (99,9%)
0,3% Carbopol ETD 2020
0,71% propoxyliertes Cocoalkylaminoxid^{*)} mit 10 PO-Einheiten (70 %-ig), gelöst in 8,9 % Wasser und 21,1 % Ethanol)
0,5% Hautschutzlösung (Zusammensetzung siehe Beispiel 1 bis 5)
3,49% Wasser
Viskosität: 5770 mPas

^{*)} Das als Rohstoff verwendete Cocoalkylamin hatte folgende Kohlenstoffkettenverteilung: 2 bis 6 % C₈; 4 bis 7 % C₁₀; 52 bis 59 % C₁₂; 18 bis 25 % C₁₄; 7 bis 12 % C₁₆; 1 - 3 % C₁₈; 2 - 8 % C_{18-ungesättigt}.

### Beispiel 24

Bestandteile:
85% Ethanol (99,9 %)
10% Propanol-1
0,3% Carbopol ETD 2020
0,71% propoxyliertes Cocoalkylaminoxid^{*)} mit 10 PO-Einheiten (70 %-ig), gelöst in 8,9 % Wasser und 21,1 % Ethanol)
0,5% Hautschutzlösung (Zusammensetzung siehe Beispiel 1 bis 5)
3,49% Wasser
Viskosität: 6540 mPas

^{*)} Das als Rohstoff verwendete Cocoalkylamin hatte folgende Kohlenstoffkettenverteilung: 2 bis 6 % C₈; 4 bis 7 % C₁₀; 52 bis 59 % C₁₂; 18 bis 25 % C₁₄; 7 bis 12 % C₁₆; 1 - 3 % C₁₈; 2 - 8 % C_{18-ungesättigt}.

Alle o.a. Beispiele stellen in ihrer Zusammensetzung stabile alkoholische Gele dar. Bei Einsatz der unterschiedlichen Aminoxide für die verschiedenen alkoholischen Gemische werden Gele mit etwa den gleichen Viskositätseigenschaften erhalten. Dabei liegen die Konzentrationsverhältnisse (Verdicker und Coverdicker) bei den unterschiedlichen Kombinationen in den gleichen Größenordnungen. Die Aminoxide (Coverdicker) bilden jeweils mit der hochverzweigten Polyacrylsäure stark strukturierte Systeme.

## Patentansprüche

1. Alkoholische Desinfektionsgele, **dadurch gekennzeichnet, daß** sie ein Verdickungssystem enthalten, welches aus mindestens einer verzweigten Polyacrylsäure und einem oder mehreren Aminoxiden als Co-Verdicker besteht,
• wobei der oder die Alkohole gewählt werden aus der Gruppe Ethanol, Propanol-1 und/oder Propanol-2 (entweder einzeln oder in beliebigen Mischungen miteinander) und
• wobei die Gele frei sind von dem (74 : 6 : 20)-Copolymerisat von Isooctylacrylat : Acrylamide : Vinylacetat.

2. Alkoholische Gele nach Anspruch 1, **dadurch gekennzeichnet, daß** als Aminoxide Stoffe der allgemeinen Strukturformel (I) gewählt werden, 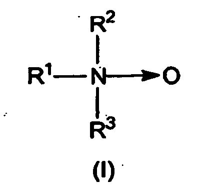 worin R¹, R² und R³ unabhängig voneinander einen aliphatischen Alkyl-, Isoalkyl- oder Alkenylrest, einen alicyclischen tertiären Alkyl- oder Amidoalkyl-Rest mit jeweils 1 bis 22 Kohlenstoffatomen oder eine Oxyalkylgruppe der Formel -(CₙH₂ₙO)ₓH bedeuten und wobei n = 2 oder 3 und x gleich ganze Zahlen von 1 bis 25 sind.

3. Alkoholische Gele nach einem der Ansprüche 1 oder 2, **dadurch gekennzeichnet, daß** der Wassergehalt der Gele 0 bis 70 Gew.-% beträgt, bezogen auf das Gesamtgewicht der Gele.

4. Alkoholische Gele nach mindestens einem der Ansprüche 1 bis 4, **dadurch gekennzeichnet, daß** der Anteil des Verdickersystems bestehend aus Polyacrylsäure und Coverdicker aus dem Bereich von 0,5 bis 5,0 Gew.-%, vorzugsweise von 0,5 bis 2,5 Gew.-% gewählt wird, jeweils bezogen auf das Gesamtgewicht der Zubereitung.

5. Alkoholische Gele nach mindestens einem der Ansprüche 1 bis 5, **dadurch gekennzeichnet, daß** das Gewichtsverhältnis von Polyacrylsäure zu Co-Verdicker von 1 : 1 bis 1 : 3 beträgt.

6. Alkoholische Gele nach mindestens einem der Ansprüche 1 bis 6, **dadurch gekennzeichnet, daß** sie 0,5 bis 7,0 Gew.-% weitere Bestandteile enthalten.

7. Alkoholische Gele nach mindestens einem der Ansprüche 1 bis 7, **dadurch gekennzeichnet, daß** sie Hautpflegestoffe enthalten.

8. Alkoholische Gele nach mindestens einem der Ansprüche 1 bis 8, **dadurch gekennzeichnet, daß** sie als weiteren Bestandteil Siliconöle, vorzugsweise Cyclomethicone enthalten.

9. Alkoholische Gele nach mindestens einem der Ansprüche 1 bis 9, **dadurch gekennzeichnet, daß** sie 0,01 bis 2,0 Gew.-% eines zusätzlichen, nicht flüchtigen antimikrobiellen Wirkstoffes enthalten.

10. Alkoholische Gele nach mindestens einem der Ansprüche 1 bis 10, **dadurch gekennzeichnet, daß** sie eine Viskosität von 1.000 bis 50.000 mPa·s aufweisen.

11. Verwendung von alkoholischen Gelen nach einem der Ansprüche 1 bis 10 zur Händedesinfektion.

12. Verwendung von Aminoxiden der Formel (I) 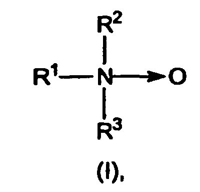 worin R¹, R² und R³ unabhängig voneinander einen aliphatischen Alkyl-, Isoalkyl- oder Alkenylrest, einen alicyclischen tertiären Alkyl- oder Amidoalkyl-Rest mit jeweils 1 bis 22 Kohlenstoffatomen oder eine Oxyalkylgruppe der Formel -(CₙH₂ₙO)ₓH bedeuten und wobei n = 2 oder 3 und x gleich ganze Zahlen von 1 bis 25 sind, als Coverdicker in Kombination mit verzweigter Polyacrylsäure zu Herstellung alkoholischer Gele

## Claims

1. Alcoholic disinfectant gels, **characterized** thereby that they contain a thickening system which comprises at least one branched polyacrylic acid and one or more amine oxides as a co-thickener,
• whereby the alcohol or alcohols are selected from the group ethanol, propanol-1 and/or propanol-2 (either individually or in any mixtures with each other) and
• whereby the gels are free of the (74:6:20) copolymer of isooctyl acrylate : acrylamide : vinyl acetate.

2. Alcoholic gels according to Claim 1, **characterized** thereby that the following are selected as amine oxide agents of the general structural formula (I), 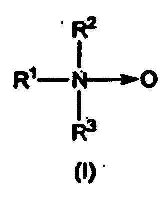 where R¹, R² and R³ represent, independently of one another, an aliphatic alkyl-, isoalkyl- or alkenyl radical, an alicyclic tertiary alkyl- or amidoalkyl radical with 1 to 22 carbon atoms respectively or an oxyalkyl group of the formula -(CₙH₂ₙ O)ₓH and where n = 2 or 3 and x is equal to whole numbers from 1 to 25.

3. Alcoholic gels according to one of Claims 1 or 2, **characterized** thereby that the water content of the gels is between 0 and 70% by weight, in relation to the total weight of the gels.

4. Alcoholic gels according to at least one of Claims 1 to 4, **characterized** thereby that the content of the thickening system consisting of polyacrylic acid and co-thickener is selected from the range between 0.5 to 5.0% by weight, preferably between 0.5 to 2.5% by weight, in relation to the total weight of the preparation respectively.

5. Alcoholic gels according to at least one of Claims 1 to 5, **characterized** thereby that the weight ratio of polyacrylic acid to co-thickener is between 1:1 and 1:3.

6. Alcoholic gels according to at least one of Claims 1 to 6, **characterized** thereby that they contain 0.5 to 7.0% by weight of further components.

7. Alcoholic gels according to at least one of Claims 1 to 7, **characterized** thereby that they contain skincare agents.

8. Alcoholic gels according to at least one of Claims 1 to 8, **characterized** thereby that they contain silicone oils, preferably cyclomethicones, as a further component.

9. Alcoholic gels according to at least one of Claims 1 to 9, **characterized** thereby that they contain 0.01 to 2.0% by weight of an additional, non-volatile antimicrobial active substance.

10. Alcoholic gels according to at least one of Claims 1 to 10, **characterized** thereby that they exhibit a viscosity of 1,000 to 50,000 mPa.s.

11. Use of alcoholic gels according to one of Claims 1 to 10 for disinfection of the hands.

12. Use of amine oxides of the formula (I) 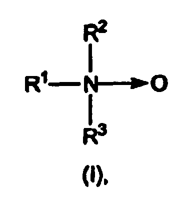 where R¹, R² and R³ represent, independently of one another, an aliphatic alkyl-, isoalkyl- or alkenyl radical, an alicyclic tertiary alkyl- or amidoalkyl radical with 1 to 22 carbon atoms respectively or an oxyalkyl group of the formula -(CₙH₂ₙO)ₓH and where n = 2 or 3 and x is equal to whole numbers from 1 to 25, as a co-thickener in combination with branched polyacrylic acid for the production of alcoholic gels.

## Revendications

1. Gel désinfectant à base d'alcool, **caractérisé en ce qu'**il comprend un système épaississant, comprenant au moins un acide polyacrylique à chaîne ramifiée et un ou plusieurs aminoxyde(s) jouant le rôle d'un co-épaississant,
• moyennant quoi le ou les alcool(s) est / sont choisi(s) dans le groupe comprenant l'éthanol, le propanol-1 et / ou le propanol-2 (utilisés individuellement ou sous la forme d'un mélange de ces alcools) et
• moyennant quoi le gel est exempt du copolymère (74:6:20) d'isooctyl - acrylate: acrylamide: acétate vinyle.

2. Gel à base d'alcool selon la revendication 1, **caractérisé en ce que** l'aminoxyde est choisi parmi les substances de formule générale (I), 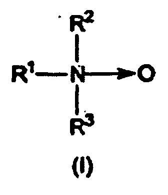 dans laquelle R¹, R² et R³ représentent, indépendamment l'un de l'autre, un radical alkyl, isoalkyl ou alkényl aliphatique, un radical amidoalkyl, ou alkyl tertiaire alicyclique comprenant chacun entre 1 et 22 atomes de carbone ou un groupe oxyalkyl de la formule (CₙH₂ₙO)ₓH et dans laquelle n = 2 ou 3 et x représente un nombre entier compris entre 1 et 25.

3. Gel à base d'alcool selon l'une quelconque des revendications 1 et 2, **caractérisé en ce que** la teneur en eau du gel est comprise entre 0 et 70 % en poids, d'après le poids total du gel.

4. Gel à base d'alcool selon au moins l'une quelconque des revendications 1 à 4, **caractérisé en ce que** la teneur en système épaississant comprenant de l'acide polyacrylique et un co-épaississant est compris entre 0,5 et 5,0 % en poids, et préférentiellement entre 0,5 et 2,5 % en poids, d'après le poids total de la préparation.

5. Gel à base d'alcool selon au moins l'une quelconque des revendications 1 à 5, **caractérisé en ce que** le rapport de poids de l'acide polyacrylique par rapport au co-épaississant est compris entre 1 : 1 à 1 : 3.

6. Gel à base d'alcool selon au moins l'une quelconque des revendications 1 à 6, **caractérisé en ce qu'**il comprend entre 0,5 et 7,0 % en poids d'autres substances.

7. Gel à base d'alcool selon au moins l'une quelconque des revendications 1 à 7, **caractérisé en ce qu'**il comprend des substances à effet protecteur de la peau.

8. Gel à base d'alcool selon au moins l'une quelconque des revendications 1 à 8, **caractérisé en ce que** les autres substances qu'il comprend sont des huiles de silicone, et préférentiellement du cyclométhicone.

9. Gel à base d'alcool selon au moins l'une quelconque des revendications 1 à 9, **caractérisé en ce qu'**il comprend entre 0,01 et 2,0 % en poids d'un autre principe actif antimicrobien non volatil.

10. Gel à base d'alcool selon au moins l'une quelconque des revendications 1 à 10, **caractérisé en ce qu'**il présente une viscosité de 1,000 à 50,000 mPa-s.

11. Utilisation d'un gel à base d'alcool selon l'une quelconque des revendications 1 à 11 pour la désinfection de la peau .

12. Utilisation d'aminoydes de la formule (I) 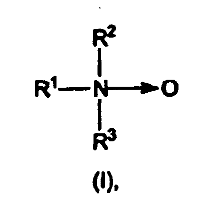 dans laquelle R¹, R² et R³ représentent, indépendamment l'un de l'autre, un radical alkyl, isoalkyl ou alkényl aliphatique, un radical amidoalkyl, ou alkyl tertiaire alicyclique comprenant chacun entre 1 et 22 atomes de carbone ou un groupe oxyalkyl de la formule (CₙH₂ₙ O)ₓH et dans laquelle n = 2 ou 3 et x représente un nombre entier compris entre 1 et 25, en tant que co-épaississant en association avec un acide polyacrylique à chaîne ramifiée pour la fabrication d'une gelée à base d'alcool.
